(19)

Europäisches
Patentamt
European
Patent Office
Office européen
des brevets

(11)  **EP 4 510 139 A1**

(12)  **EUROPEAN PATENT APPLICATION**
published in accordance with Art. 153(4) EPC

(43) Date of publication:
**19.02.2025  Bulletin 2025/08**

(21) Application number: **24741704.1**

(22) Date of filing: **10.01.2024**

(51) International Patent Classification (IPC):
*G16C 20/40* (2019.01)      *G16C 20/20* (2019.01)
*G16C 20/70* (2019.01)      *G16C 20/80* (2019.01)
*G16C 20/90* (2019.01)

(52) Cooperative Patent Classification (CPC):
**G16C 20/20; G16C 20/40; G16C 20/70;**
**G16C 20/80; G16C 20/90**

(86) International application number:
**PCT/KR2024/000498**

(87) International publication number:
**WO 2024/151083 (18.07.2024 Gazette 2024/29)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **11.01.2023  KR 20230004075**
**11.01.2023  KR 20230004076**
**09.01.2024  KR 20240003693**

(71) Applicant: **LG Chem, Ltd.**
**Yeongdeungpo-gu**
**Seoul 07336 (KR)**

(72) Inventors:
• **JO, Seong In**
**Daejeon 34122 (KR)**

• **LEE, Kyu Hwang**
**Daejeon 34122 (KR)**
• **KIM, Jae Hyun**
**Daejeon 34122 (KR)**
• **PARK, Sae Bo Mi**
**Daejeon 34122 (KR)**
• **KIM, Eun Hee**
**Daejeon 34122 (KR)**
• **OH, Jeong Min**
**Daejeon 34122 (KR)**
• **BAE, Yong Jin**
**Daejeon 34122 (KR)**

(74) Representative: **Hoffmann Eitle**
**Patent- und Rechtsanwälte PartmbB**
**Arabellastraße 30**
**81925 München (DE)**

(54)  **METHOD AND SYSTEM FOR DETERMINING SIMILARITY BY COMPARING 1H-NMR AND 1H-1H COSY NMR SPECTRA**

(57)  The invention relates to a system for estimating a molecular structure by verifying structural features included in a molecular structure as structural features of a candidate molecular structure, comparing an NMR spectrum of a target substance with spectra of candidates generated from the verified candidate molecular structure to determine a similar candidate molecular structure based on the spectrum, and when calculating similarity, by using 2D spectrum information such as COSY information rather than 1D spectrum information, molecular structure accuracy of an aromatic substance is provided.

EP 4 510 139 A1

**FIG. 2**

**Description**

## TECHNICAL FIELD

The present invention relates to a technique for enabling a researcher to efficiently estimate the molecular structure of an unknown substance by calculating a plurality of candidate molecular structures and extracting unsuitable candidate molecular structures from the plurality of candidate molecular structures to reduce the number of candidate molecular structures.

In addition, the present invention relates to a technique for extracting a final candidate molecular structure from the reduced number of candidate molecular structures through 1H-NMR spectrum analysis and comparison.

## BACKGROUND ART

1H-NMR spectrum analysis is performed to estimate the molecular structure of an unknown substance. At this time, an analyst derives all possible candidate molecular structures from an acquired 1H-NMR spectrum, and predicts peak positions and shapes of all hydrogens in each candidate molecular structure to create a virtual spectrum. Thereafter, the most reasonable structure is selected by comparing the virtual spectrum with the actual spectrum (in terms of notation, in the present invention, 1H-NMR and $^1$H-NMR, 1H-1H NMR and $^1$H-$^1$H COSY NMR are regarded the same, respectively).

However, when a large number of peaks are concentrated in a small region, as in the case of an aromatic macromolecule, there is a problem in that the above-described method is difficult for use in comparing similar molecules.

For example, the following prior art does not use information such as peak multiplicity in a spectrum, and thus, is not capable of reflecting detailed structural information, which results in no deriving an accurate molecular structure estimation result.

A Structure Elucidation System Based on $^1$H NMR and H-H COSY Spectra in Organic Chemistry, Hideyuki Masui and Huixiao Hong, J. Chem. Inf. Model. 2006, 46, 2,\

## DISCLOSURE OF THE INVENTION

## TECHNICAL PROBLEM

The present invention aims to solve the problem of the prior art and to provide a method and a system capable of performing accurate 1H-NMR spectrum analysis even for a substance having a large molecular size or a complex shape, such as an aromatic ring.

## TECHNICAL SOLUTION

In order to solve the above-mentioned technical problem, the present invention provides a method for predicting a molecular structure of a target substance from a 1H-NMR spectrum of the target substance, wherein the method includes a key spectrum acquisition procedure for acquiring a 1H-NMR spectrum of a target substance, a candidate spectrum information acquisition procedure for acquiring spectrum information of a virtual spectrum of each candidate substance from candidate molecular structures of the target substance as candidate spectrum information, a spectrum comparison procedure for comparing the key spectral information with the candidate spectrum information to calculate similarity between the key spectrum and the candidate spectrum; and a target substance molecular structure determination procedure for deriving a candidate spectrum with the highest similarity in the spectrum comparison procedure, and selecting the molecular structure of a corresponding candidate substance as the molecular structure of the target substance.

In addition, the present invention provides a method for determining similarity by comparing two NMR spectra, wherein the method includes a peak value information extraction step of extracting peak value information for spectrum comparison from each peak of a first spectrum as a key spectrum and a second spectrum as a candidate spectrum, a peak correspondence step of matching each peak of the first spectrum and the second spectrum; a spectrum similarity calculation step of calculating similarity between the first spectrum and the second spectrum by comparing the peak value information of the peaks of the first spectrum and the second spectrum matched.

Furthermore, the present invention provides a system for estimating a molecular structure of a target substance, wherein the system includes a spectrum acquisition device that acquires a 1H-NMR spectrum and a 1H-1H NMR spectrum of a target substance from an NMR device as a key spectrum of the target substance, a candidate molecular structure acquisition module that acquires candidate molecular structures of the target substance and calculates a candidate molecular structure list, a virtual spectrum calculation module that calculates a virtual spectrum as a candidate spectrum from the candidate molecular structures included in the candidate molecular structure list, a spectrum information extraction module that extracts spectrum information from each of the key spectrum and the candidate spectrum, a spectrum similarity calculation module that calculates similarity between the key spectrum and the candidate spectrum by using the spectrum information of the key spectrum and the candidate spectrum, a final estimated structure output module that outputs a candidate molecular structure corresponding to a candidate spectrum with the highest similarity as a final estimated molecular structure of the target substance.

In addition, the present invention extracts hydrogen structural features that identify hydrogens having a predetermined structural feature from the candidate molecular structures, and through verification of suitability of candidate molecular structures, which includes a procedure of verifying whether all hydrogens having the predetermined structural feature may be assigned to all peaks of the 1H-NMR spectrum, verifies each candidate molecular structure to update a candidate molecular structure list excluding unsuitable candidate molecular structures, thereby reducing the number of candidate molecular structures to increase computational efficiency.

To this end, a system for estimating a molecular structure of a target substance according to the present invention includes a hydrogen structural feature identification module of identifying hydrogen atoms having a predetermined structural feature in each candidate molecular structure of the target substance, a peak assignment validity determination module that verifies, based on the structural feature information of each hydrogen identified from the candidate molecular structure, whether each hydrogen of the candidate molecular structure may be assigned to all peaks in the spectrum, a valid candidate molecular structure output module that updates the candidate molecular structure list by using results of the peak assignment validity determination module.

## ADVANTAGEOUS EFFECTS

The present invention provides a method for calculating similarity of two spectra by using 2D NMR spectrum information as well as 1D NMR spectrum information to bring an effect of accurately and efficiently estimating a molecular structure of a target substance by comparing a spectrum of the target substance and a spectrum of a candidate substance.

In addition, the present invention quickly excludes unsuitable candidate substances from candidate molecular structures, and thus, brings an effect of estimating an accurate molecular structure of an unknown substance more quickly through similarity calculation.

## BRIEF DESCRIPTION OF THE DRAWINGS

The following drawings attached herein illustrate preferred embodiments of the present invention by example, and with the above-described detailed description of the present invention, serve to enable technical concepts of the present invention to be further understood, and therefore, the present invention should not be construed as limited to only the matters described in such drawings.

FIG. 1 is an example of a COSY spectrum used in the present invention.
FIG. 2 is a flowchart showing a procedure for calculating spectrum similarity and estimating a molecular structure according to the present invention.
FIG. 3 is a block diagram of a system for calculating spectrum similarity and estimating a molecular structure of the present invention.

## MODE FOR CARRYING OUT THE INVENTION

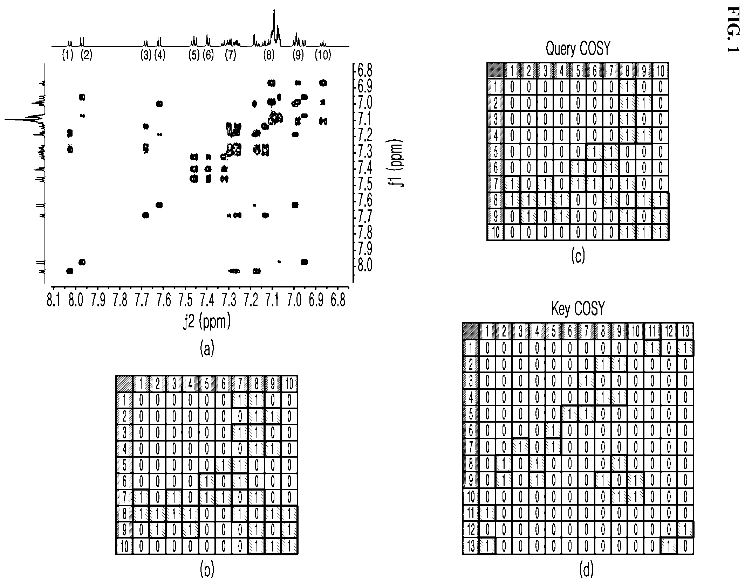

. The present invention uses a 1H-1H COSY spectrum as one of a 1H-NMR spectrum and a 2D NMR spectrum of a substance. The 1H-1H COSY spectrum is obtained from a nuclear magnetic resonance spectrometer (NMR). Both a horizontal axis and a vertical axis are composed of a 1H spectrum in the 1H-1H COSY spectrum, and FIG. 1 shows an example thereof. In the present invention, a 1H-NMR spectrum, a $^1$H-NMR spectrum, a 1H-1H COSY NMR spectrum, and a $^1$H-$^1$H COSY NMR spectrum each denote the same thing.

1. Method for estimating molecular structure of target substance according to the present invention

A method for estimating the molecular structure of a target substance according to the present invention is an invention which acquires spectrum (this is called a virtual spectrum) information corresponding to a plurality of candidate substance molecular structures, and then compares virtual spectrum information of a candidate substance with spectrum (this is called a key spectrum) information of a target substance to select a virtual spectrum that is most similar to the key spectrum, and selects the molecular structure of the corresponding candidate substance as the molecular structure of the target substance.

The procedure according to the present invention is largely composed of the following three procedures. First, A.

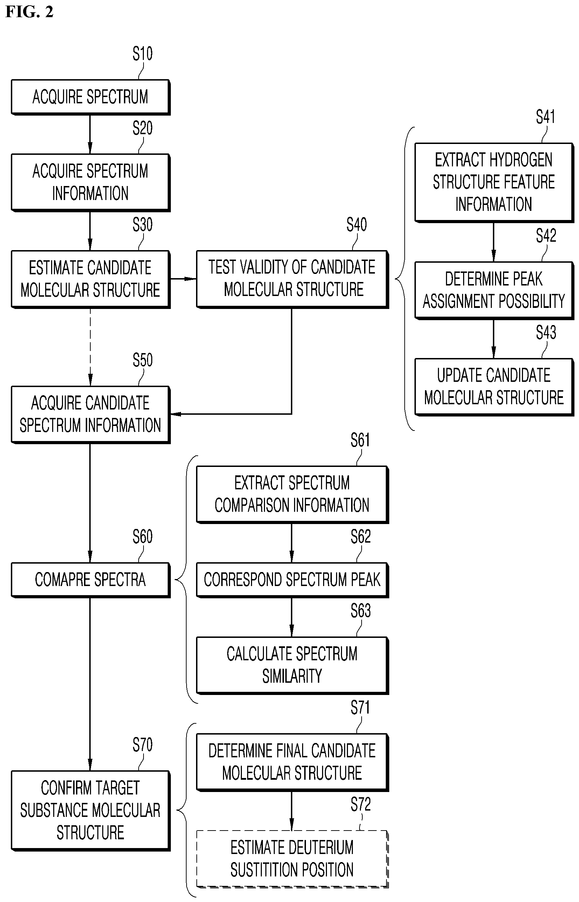

a procedure of acquiring the spectrum information of a target substance for which the molecular structure is to be estimated, followed by B. a procedure of selecting candidate molecular structures of the target substance, and then C. a procedure of comparing virtual spectra of the candidate molecular structures with a spectrum of the target substance to acquire a final molecular structure of the target substance from the candidate molecular structures. Hereinafter, each procedure will be described in more detail with reference to FIG. 2.

A. Procedure of acquiring spectrum information of target substance

(1) Key spectrum acquisition step (S10)

This is a procedure of acquiring a 1H-NMR spectrum and a 1H-1H COSY spectrum of a target substance from an NMR. The 1H-NMR spectrum and the 1H-1H COSY spectrum acquired are called a key spectrum.

(2) Key spectrum information acquisition step (S20)

Spectrum information is acquired from the key spectrum. The spectrum information includes the following information.

(a) Peak position (chemical shift value): x-axis value of each peak in one-dimensional spectrum
(b) Peak split value (multiplicity (split)): Number of small peaks forming each peak (for example, in the spectrum of FIG. 1, the split of the leftmost peak is 2, and the split of the rightmost peak is 3)
(c) Peak integral value: Area value of each peak
(d) COSY information (COSY): Information of all peak pairs in which COSY peaks are observed.

Example) In the two-dimensional key spectrum of FIG. 1, a red dot is displayed at a peak position related to the first peak on the left, and this is called a COSY peak. Since the first peak showed a COSY peak with the seventh and eighth peaks, for a matrix with rows and columns that are respectively a peak list, an element at a (1, 7), (1, 8) position is set to 1.
FIG. 1 is a diagram showing a predetermined two-dimensional key spectrum (a) and a COSY matrix (b) displaying information of a COSY peak thereof, and in (a) of FIG. 1, if there is a COSY peak present between two 1D peaks, an element value of the COSY matrix of (b) in FIG. 1 is set to 1. In the example above, since the first peak (1) shows the COSY peaks with the seventh and eighth peaks (7) and (8), it can be seen that the element values of COSY (1, 7) and COSY (1, 8) are each set to 1 in the COSY matrix of FIG. 1 (b).

B. Procedure of selecting candidate molecular structures of target substance

A procedure of selecting candidate molecular structures of the target substance will be described.

(1) Procedure of acquiring candidate molecular structures (S30)

This is a step of acquiring candidate molecular structure information of the target substance. A candidate molecular structure is estimating a molecular structure of a candidate that the target substance may have, in order to finally estimate what kind of molecular structure the target substance has. By using measurement information of the target substance, such as mass spectrometry for the target substance, it is possible to estimate information of a plurality of candidate molecular structures, which could be the molecular structure of the target substance.
The plurality of candidate molecular structures are acquired by an analysis method such as LC-MS/MS, and for example, a candidate molecular structure may be acquired by acquiring a molecular weight and a molecular formula of the target substance, and then searching a molecular structure having the same molecular formula in a known substance database (SCIFinder, PubChem, etc.). Another example is a method for creating possible candidate molecular structures by acquiring partial structure information of a molecule from MS/MS analysis results and then assembling the partial structure information.
A list of candidate molecular structures including the plurality of candidate molecular structures of the target substance acquired as described above is created.

(2) Procedure of testing validity of candidate molecular structures (S40)

A molecular structure of the target substance may be acquired through spectrum comparison of each spectrum of the above-acquired candidate molecular structures of the target substance according to a procedure of acquiring a final molecular structure of the target substance from the candidate molecular structures, which is described below in Procedure C.

However, in this case, spectra of numerous candidate molecular structures should be compared, so that in order to reduce the number of candidate molecular structures, a procedure for verifying suitability of candidate molecular structures may be performed prior to the spectrum comparison procedure.

The candidate molecular structure validity procedure extracts feature information of hydrogen from a plurality of candidate molecular structures, determines validity of non-suitable candidate molecular structures based on whether all hydrogens of each candidate molecular structure may be assigned to all peaks in a spectrum of the target substance, and excludes the non-suitable candidate molecular structures from the list of candidate molecular structures. This procedure follows detailed procedures below.

(2-1) Hydrogen structural feature information extraction step (S41)

This is a step of identifying structural feature information of hydrogens constituting each candidate molecular structure included in the list of candidate molecular structures to extract hydrogen feature information of each candidate molecular structure. Hydrogen feature information to be identified includes the following:

① Number of isomorphic hydrogen
It refers to the number of hydrogens having a bonding structure that is structurally isomorphic with that of a target hydrogen. The number of isomorphic hydrogens becomes an integral value of a corresponding hydrogen peak.
② Number of hydrogens linked to 1st-neighbor atom
It is the number of hydrogens linked to a 1st-neighbor atom among atoms linked to the target hydrogen. Multiplicity of the target hydrogen is determined from the number of 1st-neighbor atom-linked hydrogens linked to the 1st-neighbor atom.
(3) Number of hydrogens linked to 2nd-neighbor atom
It is the number of hydrogens linked to a second linked atom via the 1st-neighbor atom among atoms linked to the target hydrogen. That is, it is the number of hydrogens linked to an atom directly linked to the 1st-neighbor atom. This affects the multiplicity of the target hydrogen, and is used to predict the shape of a peak.
④ Other hydrogens that may have COSY peak
Other hydrogens that may have the same COSY peak as that of the target hydrogen are identified. If the distance between two hydrogens is within a specific bonding distance, a COSY signal is displayed.

As such, by extracting the structural feature information of each hydrogen from each candidate molecular structure, it is determined whether it is possible to assign each hydrogen of each candidate molecular structure to each peak of the key spectrum of the target substance based on the feature information of the hydrogen of each candidate molecular structure in the next step.

(2-2) Peak assignment possibility determination step (S42)

This is a step of determining whether all hydrogens of a given candidate molecular structure may be assigned to all peaks of the key spectrum.

If all hydrogens of the given candidate molecular structure cannot be assigned to all peaks in the key spectrum, a molecular structure validity test according to the present invention determines that the corresponding candidate molecular structure is an invalid molecular structure.

Therefore, in order to determine if assignment is impossible, all possible assignment methods should be reviewed, which requires a very large amount of calculation. For example, if a predetermined candidate molecular structure includes m distinguishable hydrogens, and there are n peaks in a spectrum, there are mPn possible assignment methods, so that the time to determine assignment suitability increases exponentially according to molecular complexity.

Accordingly, the present invention determines the possibility of assignment by replacing a problem of assigning all hydrogens of the candidate molecular structure to each peak of the key spectrum with a mixed integer linear programming (MIP) problem having linear constraints.

Here, since there is no optimization target for the MIP problem, slack variables, s, and t are introduced, and the possibility of assignment is determined based on the success/failure of optimization itself.

An equation for optimization is Equation 1 below, and the optimization is to obtain optimal solutions si and ti that minimize Equation 1.

[Equation 1]

$$minimize \sum_{i}^{N} (s_i + t_i)$$

for $M \times N$ sized matrix X

(If hydrogen j is not assigned to peak i, $X_{ij}=0$, if hydrogen j is assigned to peak i, $X_{ij}=1$, j=1, 2, ..., M, i=1, 2, ..., N)

<Constraints>

Constraints for the calculation of Equation 1 are as follows.

① $s_i \geq 0$, $t_i \geq 0$ for $1 \leq i \leq N$

② For all $1 \leq i \leq N$, the peak integral value of hydrogen j is $intg_{hydrogen}(j)$, and the integral value of peak i of the key spectrum is $intg_{peak}(i)$,

$$s_i = intg_{peak}(i) - \sum_{j}^{M} X_{ij} \cdot intg_{hydrogen}(j)$$

$$t_i = -intg_{peak}(i) + \sum_{j}^{M} X_{ij} \cdot intg_{hydrogen}(j)$$

③ $X_{ij} = 0$, (if the multiplicity of hydrogen j is different from the multiplicity of peak i)

④ $X_{ij} = 0$, (if the peak integral value of hydrogen j is greater than the integral value of peak i)

(5) For two peaks i_1 and i_2 having COSY peaks, there must be at least one COSY peak present between all hydrogen sets C_hydrogen(i1) assigned to the i_1 and all hydrogen sets C_hydrogen(i2) assigned to the i_2.

(2-3) Candidate molecular structure list update step (S43)

In the optimized Equation 1, if the optimization is successful and the optimal solutions for s and t are obtained, all hydrogen of the corresponding structure may be assigned to peaks of the key spectrum, and this is determined to be a valid structure. If the optimization fails and no optimal solution is obtained, it is considered that there are one or more unassignable hydrogens present, so that it is determined that the corresponding candidate structure is a candidate molecular structure not suitable for a given spectrum.

In this step, as such, unsuitable candidate molecular structures are excluded and the list of candidate molecular structures is updated to output a list of valid candidate molecular structures.

C. Final molecular structure selection step

This is a procedure of comparing spectrum information of candidate molecular structures included in the obtained candidate molecular structure list or in the valid candidate molecular structure list with the spectrum information of the target substance to select the final molecular structure of the target substance. This is accomplished through the detailed procedures below.

(1) Candidate spectrum information acquisition procedure (S50)

A virtual spectrum corresponding to each candidate molecular structure is acquired from the above-acquired information of the plurality of candidate molecular structures, which is set as a spectrum of each candidate molecular structure, and information of each spectrum is acquired. The virtual spectrum of each candidate molecular structure is acquired using a known virtual spectrum information calculation device or software for calculating virtual spectrum information when molecular structure information is input.

Candidate spectrum information, which is spectrum information of a candidate spectrum for a candidate molecular structure to be obtained, includes items such as the key spectrum information described above.

(2) Spectrum comparison procedure (S60)

By comparing the spectrum information (each peak value data) of the key spectrum, which is the spectrum of the target substance, with the spectrum information of each candidate, similarity between the key spectrum and the candidate spectrum is calculated. A spectrum comparison method according to the present invention to be described below is applied to the spectrum comparison.

A virtual spectrum of a candidate substance, which is most similar to the key spectrum, is derived according to a spectrum similarity calculation procedure to be described below.

A procedure of determining similarity by comparing NMR spectra of two molecules according to the present invention will be described.

(2-1) Spectrum comparison information extraction from key spectrum and candidate spectrum (S61)

Comparison information for spectrum comparison is extracted from each peak of the key spectrum and the candidate spectrum. The comparison information is the spectrum information described above.

(2-2) Peak correspondence step of matching peak of candidate spectrum to peak of key spectrum (S62)

This is a step of assigning a peak Qi of a candidate spectrum to a peak Ki of the key spectrum. Here, the i is a peak number of the key spectrum and the candidate spectrum.

When matching the Qi to the Ki, the following rules should be satisfied.

① An integral value of Qi should not be greater than an integral value of Ki.
② A split value of Qi should be the same as a split value of Ki.
③ One or more hydrogens having a COSY relationship with Qi should be assigned to other peaks having a COSY relationship with Ki.

COSY peak assignment of the query peak Qi and the key peak Ki will be described with reference to (c) and (d) of FIG. 1.

If a cosy matrix of the key spectrum and a cosy matrix of the query spectrum are obtained as shown in (c) and (d) of FIG. 1, the constraints mean that when there is a COSY peak between the key peaks i1 and i2, there must be at least one reciprocal COSY peak present between a query peak set C_query(i1) assigned to i 1 and a query peak set C_query(i2) assigned to i2.

Considering a case in which two of K11 and K13 are assigned to a Q7 peak, and three of K8, K9, and K10 are assigned to a Q8 peak, in (c) of FIG. 1, the Q7 peak and Q8 peak have a COSY peak (a matrix element is 1), so that there should be a COSY peak present between peaks assigned to Q7 and peaks assigned to Q8. However, referring to (d) of FIG. 1, there is no COSY peak present between K11 and K13 groups and K8, K9, and K10 groups, such assignment is an unsuitable assignment.

(2-3) Similarity calculation step of two spectra (S63)

According to the above-described procedure, peaks Qi (i = 1, 2, ..., M) (referred to as query peaks) of a candidate spectrum are assigned to peaks Kj (j = 1, 2, ..., N) (referred to as key peaks) of the key spectrum, and then similarity between the candidate spectrum and the key spectrum is calculated to derive an optimal similar candidate spectrum with the highest similarity.

The similarity is calculated by the objective function of Equation 2 below, and the optimal similar candidate spectrum with the highest similarity is one that reduces an objective function value in Equation 2.

[Equation 2]

$$f = \sum_{i}^{M} \left( \sum_{j}^{N} \left( X_{ij} \cdot \left| shift_{query}(i) - shift_{key}(j) \right|^2 \right) \right)$$

(M and N are respectively the number of peaks of the query spectrum and the key spectrum, X is a binary matrix of $M \times N$, and if the query peak Qi is not assigned to the key peak Kj, $X_{ij}=0$, and if the query peak Qi is assigned to the key peak Kj, $X_{ij}=1$. $shift_{query}(i)$ and $shift_{key}(j)$ respectively represent chemical shifts of Qi and Ki.)

Meanwhile, the calculation of the objective function has the following restrictions.

① $\sum_i^M X_{ij} \cdot intg_{query}(i) = intg_{key}(j)$ (intg$_{query}$(i) is an integral value of the query peak Qi, and intg$_{key}$(j) is an integral value of the key peak Kj)

② If the multiplicity of Kj is different from the multiplicity of Qi, $X_{ij}=0$

③ If an integral value of Qi is greater than an integral value of Kj, $X_{ij}=0$

④ For two key peaks Ki_1 and Ki_2 having a COSY peak, there must be at least one COSY peak present between a set of all query peaks C_query(Ki_1) assigned to Ki_1 and a set of all query peaks C_query(Ki_2) assigned to Ki_2.

As such, while applying the above-described constraints, similarity between two spectra is calculated by calculating the objective function according to Equation 2 above.

(3) Target substance molecular structure confirmation step (S70)

The molecular structure of a candidate substance corresponding to a candidate spectrum derived as most similar in the spectrum similarity calculation procedure is determined as the molecular structure of the target substance.

(3-1) Final candidate molecular structure determination step (S71)

A candidate spectrum with the smallest objective function calculation value to be described below is determined as an optimal similar spectrum with the highest similarity. There is a final candidate molecular structure determination step in which a candidate structure corresponding to the optimal similar spectrum is estimated as the molecular structure of the target substance. The determined final candidate molecular structure is confirmed as the molecular structure of the target substance to be estimated.

(3-2) Deuterium substitution position estimation step (S72)

The present invention may additionally include a deuterium substitution position estimation step of estimating a deuterium substitution position through spectrum comparison.

If some of hydrogens of a specific molecule are substituted with deuteriums, a 1H NMR peak of the corresponding hydrogen has a unique feature form, and if the deuterium substitution rate is not 100%, the corresponding peak appears in the form of a small peak having an integral value of a decimal point unit rather than an integer, and the corresponding peak appears in a form in which a singlet and a multiplet are mixed. That is, a 1H-NMR peak at the deuterium substitution position has a characteristic shape.

Meanwhile, it is possible to know which hydrogen of a candidate molecular structure corresponds to each peak Qi of a candidate spectrum estimated from a candidate molecular structure, and since each Qi is assigned to each Kj in the above assignment process, it is possible to know a hydrogen assigned to each Kj. Therefore, since it is possible to know a Qi assigned to a deuterium substitution peak having the characteristic shape, it is possible to know which hydrogen of the candidate molecular structure has finally been substituted with a deuterium. When performing a deuterium substitution reaction, the deuterium substitution position may be used to confirm whether the substitution has occurred at a desired position.

By applying the above principle, the deuterium substitution position estimation step finds a peak in which a singlet and a multiplet are mixed among peaks Kj of the key spectrum, finds a peak corresponding to a Qi of the optimal similar spectrum, and finds a corresponding hydrogen in the final estimated candidate molecular structure to identify the position of a hydrogen substituted with a deuterium in the molecular structure of the target substance.

2. System for estimating molecular structure of target substance according to the present invention

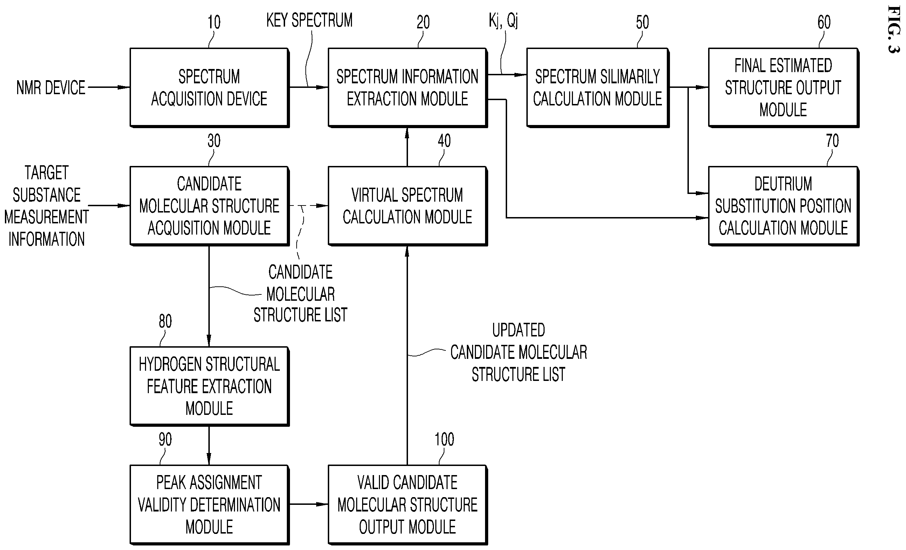

Below, with reference to FIG. 3, a molecular structure estimation system for estimating the molecular structure of a target substance according to the present invention will be described.

(1) Spectrum acquisition device (10)

This is a composition that acquires a 1H-NMR spectrum and a 1H-1HNMR spectrum of the target substance from an NMR device. It may be composed of an input unit of a computer input device that receives spectrum information from a typical NMR device. At this time, spectrum of the target substance is called a key spectrum.

(2) Spectrum information extraction module (20)

It is a composition that acquires spectrum information from the spectrum. The acquired spectrum information is the same as described in the spectrum information acquisition step. A spectrum information extraction module acquires key spectrum information including information of a key spectrum peak $Ki$ from a key spectrum, and acquires candidate spectrum information including information of a candidate spectrum peak $Qi$ of a candidate spectrum.

(3) Candidate molecular structure acquisition module (30)

It is a composition that receives results of such as mass spectrometry of the target substance and estimates and calculates a candidate molecular structure of the target substance. Candidate molecular structures, as described above, may be acquired through a method such as mass spectrometry.

In the present invention, obtaining a candidate molecular structure which may be the molecular structure of the target substance uses a known method, so that a candidate molecular structure acquisition module of the present invention may acquire a molecular weight and a molecular formula of the target substance from an LCMS/MS analyzer and search the molecular weight and the molecular formula in a known database to acquire a candidate molecular structure, or may be composed of a data input module that receives a candidate molecular structure obtained by a known method as described above.

(4) A virtual spectrum calculation module (40)

A virtual spectrum calculation module calculates a virtual spectrum for each candidate molecular structure of the target substance calculated by the candidate molecular structure acquisition module. The spectrum of each candidate molecular structure calculated by the virtual spectrum calculation module is input to the spectrum information extraction module 20 to extract candidate spectrum information. The virtual spectrum calculation module 40 is composed of a known virtual spectrum information calculation device or software that calculates virtual spectrum information when molecular structure information is input.

(5) Spectrum similarity calculation module (50)

A spectrum similarity calculation module matches the peak $Qi$ of the candidate spectrum with the peak $Kj$ of the key spectrum calculated by the spectrum information extraction module 20, and calculates similarity between the key spectrum and the candidate spectrum.

The matching method of $Kj$ and $Qi$ and the spectrum similarity calculation method are the same as described in 2. (2) and 2. (3) above. It is the spectrum with the highest similarity to a candidate spectrum for which the objective function of Equation 1 is calculated to be lowest.

(6) Final estimated structure output module (60)

A candidate structure output module determines a candidate molecular structure corresponding to the corresponding candidate spectrum from the optimal similarity spectrum calculated by the spectrum similarity calculation module 50 above as a final estimated structure for the target substance and outputs the corresponding molecular structure information.

(7) Deuterium substitution position calculation module (70)

This is a module that determines the candidate molecular structure having the optimal similarity spectrum calculated by the spectrum similarity calculation module as the molecular structure of the target substance, and identifies and calculates the position of a hydrogen substituted by a deuterium in the corresponding molecular structure.

This is to find a peak in which a singlet and multiplet are mixed among the peaks $Kj$ of the key spectrum, finds a peak corresponding to a $Qi$ of the optimal similar spectrum, and finds a corresponding hydrogen in the final estimated molecular structure of the corresponding candidate molecular structure to identify the position of a hydrogen substituted with a deuterium in the molecular structure of the target substance.

(8) Hydrogen structural feature extraction module (80)

This is a module that identifies and extracts structural feature information of hydrogen from each candidate molecular structure of the target substance calculated by the candidate molecular structure acquisition module. The structural feature information to be identified is, as described above, the number of isomorphic hydrogens, the number of hydrogens linked to the $1^{st}$-neighbor atom, the number of hydrogens linked to the $2^{nd}$-neighbor atom, and identification information for other hydrogens that may have COSY peaks.

As such, by extracting the structural feature information of each hydrogen from each candidate molecular structure, it is determined whether it is possible to assign each hydrogen of the candidate molecular structure to each peak of the spectrum based on the feature information of the hydrogen of each candidate molecular structure in the peak assignment validity determination module.

The hydrogen structural feature extraction module of the present invention may be composed of a data input module that identifies the structural feature information of hydrogen using a known algorithm for extracting hydrogen structural information or receives the hydrogen structural feature information identified using the known algorithm.

(9) Peak assignment validity determination module (90)

A peak assignment validity determination module is a module that determines, according to Equation 1 above, whether each hydrogen may be assigned to each peak of the spectrum based on the structural feature information of each hydrogen extracted above from the candidate molecular structure. The peak assignment validity determination module is composed of software that obtains an optimal solution according to Equation 1 described above.

The peak assignment validity determination module determines that if the optimal solution according to Equation 1 is not obtained, the corresponding candidate molecular structure is not suitable for the given spectrum.

(10) Valid candidate molecular structure output module (100)

The valid candidate structure output module excludes candidate molecular structures not suitable for the given spectrum as a result of verification in each of the hydrogen structural feature extraction module and the peak assignment validity determination module for the candidate molecular structures calculated above by the candidate molecular structure acquisition module, and outputs a list of the remaining candidate molecular structures as a candidate molecular structure list. That is, the valid candidate molecular structure output module updates the candidate molecular structure list by using the results of the peak assignment validity determination module.

Typically, in order to determine the suitability of a candidate molecular structure, each hydrogen position of each candidate molecular structure is compared with the shape of each peak of a spectrum to determine whether the candidate molecular structure is a valid structure, so that as a molecular structure becomes larger and more complex, the difficulty and required time increase exponentially. However, according to the present invention, by converting hydrogen, which may be distinguished by a predetermined rule, into a problem of solving an equation by optimizing only whether or not the hydrogen may correspond to each peak of an NMR spectrum of the target substance, it is possible to very quickly determine the suitability of a candidate molecular structure, and it is also possible to accurately and quickly determine the suitability for a large aromatic compound by utilizing 1H-NMR information as well as 2D 1H-1H NMR information.

In addition, typically, when comparing an actual spectrum with a virtual spectrum for molecular structure estimation of an unknown substance, a method has been used in which the similarity is evaluated higher as signals of similar intensities are positioned at similar X-axis positions. The above typical method is not accurate in determining similarity since the position and intensity of peaks are not significantly different from each other, when a large number of peaks are concentrated in a small region, as in the case of an aromatic macromolecule, and when candidate molecular structures are similar to the extent that only the substitution positions of functional groups are different.

Accordingly, the present invention compares the spectra of candidate molecular structures by utilizing not only x and y coordinates of a 1H-NMR spectrum peak, but also multiplicity and 2D 1H-1H COSY NMR information, so that it has become possible to build a system that accurately determines the similarity of candidate structures for aromatic polymers.

Meanwhile, names of the reference numerals used in the present invention are as follows.

10 Spectrum acquisition device
20 Spectrum information extraction module
30 Candidate molecular structure acquisition module
40 Virtual spectrum calculation module
50 Spectral similarity calculation module
60 Deuterium substitution position calculation module
70 Final estimated structure output module
80 Hydrogen structural feature extraction module
90 Peak assignment validity determination module 100 Valid candidate molecular structure output module.

**Claims**

1. A method for predicting the molecular structure of a target substance from a 1H-NMR spectrum and a 1H-1H COSY spectrum of the target substance, the method comprising:

a key spectrum acquisition procedure for acquiring a 1H-NMR spectrum and a 1H-1H COSY spectrum of a target substance as a key spectrum;
a procedure for acquiring key spectrum information including a peak position, a peak split value, a peak integral value, and cosy information of the key spectrum;
a candidate molecular structure acquisition procedure for acquiring molecular structure information of a plurality of candidates of the target substance;
a procedure for creating a candidate molecular structure list including the acquired molecular structure information of the plurality of candidates;
a candidate spectrum information acquisition procedure for acquiring a virtual spectrum corresponding to each candidate molecular structure from a plurality of candidate molecular structures in the candidate molecular structure list as a spectrum of each candidate molecular structure and acquiring the information thereof;
a spectrum comparison procedure for comparing the key spectral information with the candidate spectrum information to calculate similarity between the key spectrum and the candidate spectrum; and
a target substance molecular structure determination procedure for deriving a candidate spectrum with the highest similarity in the spectrum comparison procedure, and selecting the molecular structure of a corresponding candidate substance as the molecular structure of the target substance.

2. The method of claim 1, further comprising,

after the procedure for creating a candidate molecular structure list,
a hydrogen structural feature information extraction step of extracting structural feature information of each hydrogen from the candidate molecular structures of the target substance;
a peak assignment possibility determination step of verifying whether all hydrogens of each candidate molecular structure may be assigned to all peak positions of the key spectrum of the target substance, based on the structural feature information of each hydrogen; and
a candidate molecular structure list updating step of leaving only the candidate molecular structures which may assign all of the hydrogens to all peak positions of the key spectrum and excluding the remaining candidate molecular structures from the candidate molecular structure list.

3. The method of claim 2, wherein the peak assignment possibility determination step obtains an optimal solution of Equation 1, and if the optimal solution is obtained, determines the same as a candidate molecular structure, and the candidate molecular structure list updating step updates the candidate list by excluding unsuitable candidate molecular structures:

$$(\text{Equation 1})$$

$$minimize \sum_{i}^{N} (s_i + t_i)$$

for $M \times N$ sized matrix X
If hydrogen j is not assigned to peak i, $X_{ij}=0$, if hydrogen j is assigned to peak i, $X_{ij}=1$, j=1, 2, ..., M, i=1, 2, ..., N,

<Constraints>

① $s_i \geq 0$, $t_i \geq 0$ for $1 \leq i \leq N$
② For all $1 \leq i \leq N$, the peak integral value of hydrogen j is $intg_{hydrogen}(j)$, and the integral value of peak i of the key spectrum is $intg_{peak}(i)$,

$$s_i = intg_{peak}(i) - \sum_{j}^{M} X_{ij} \cdot intg_{hydrogen}(j)$$

$$t_i = -intg_{peak}(i) + \Sigma_{j}^{M} X_{ij} \cdot intg_{hydrogen}(j)$$

③ $X_{ij}$ = 0, if the multiplicity of hydrogen j is different from the multiplicity of peak i

④ $X_{ij}$ = 0, if the peak integral value of hydrogen j is greater than the integral value of peak i

(5) For two peaks i_1 and i_2 having COSY peaks, there must be at least one COSY peak present between all hydrogen sets C_hydrogen(i1) assigned to the i_1 and all hydrogen sets C_hydrogen(i2) assigned to the i_2.

4. The method of any one of claim 1 to claim 3, wherein the key spectrum information and the candidate spectrum information comprise:

a COSY peak, which is a pair of each peak of each spectrum and another peak that is related to the peak;
a peak integral value, which is the area of each peak;
a position value of each peak; and
a peak split value, which is the number of small peaks constituting each peak.

5. The method of claim 4, wherein the spectrum comparison procedure comprises, a peak correspondence procedure of assigning, when a peak of the key spectrum is Kj and a peak of the candidate spectrum is Qi, the peaks to satisfy Equation 2 below:

(Equation 2)

Integral value of Qi $\leq$ Integral value of Kj

Split value of Qi $=$ Split value of Kj

6. The method of claim 5, wherein the peak correspondence procedure corresponds such that one or more hydrogens having a COSY relationship with Qi are assigned to another peak having a COSY relationship with Ki.

7. The method of claim 6, wherein the target substance molecular structure determination step comprises:

a final candidate molecular structure determination step of determining a molecular structure corresponding to a candidate spectrum derived as most similar to the key spectrum among the candidate spectra as the molecular structure of the target substance; and
a deuterium substitution position estimation step of identifying hydrogen of an estimated molecular structure of the target substance corresponding to a peak in which a singlet and a multiplet are mixed among the peaks of the key spectrum as a hydrogen substituted with a deuterium.

8. A method for determining similarity by comparing two NMR spectra, the method comprising:

a peak value information extraction step of extracting peak value information for spectrum comparison from each peak of a first spectrum as a key spectrum and a second spectrum as a candidate spectrum;
a peak correspondence step of matching each peak of the first spectrum and the second spectrum;
a spectrum similarity calculation step of calculating similarity between the first spectrum and the second spectrum by comparing the peak value information of the peaks of the first spectrum and the second spectrum matched.

9. The method of claim 8, wherein the peak value information comprises:

a COSY peak, which is a pair of a corresponding peak and another peak that is related to the peak;
a peak integral value, which is the area of each peak;
a position value of each peak; and

a peak split value, which is the number of small peaks constituting each peak.

10. The method of claim 9, wherein the peak correspondence step matches each peak to satisfy Equation 3 below:

$$(\text{Equation 3})$$

$$\text{Integral value of Qi} \leq \text{Integral value of Kj}$$

$$\text{Split value of Qi} = \text{Split value of Kj}$$

11. The method of claim 9, wherein in the spectrum similarity calculation step, the similarity is calculated by an objective function of Equation 4 below:

$$(\text{Equation 4})$$

$$f = \sum_{i}^{M} \left( \sum_{j}^{N} \left( X_{ij} \cdot \left| \text{shift}_{\text{query}}(i) - \text{shift}_{\text{key}}(j) \right|^{2} \right) \right)$$

M and N are respectively the number of peaks of the query spectrum and the key spectrum, X is a binary matrix of $M \times N$, and if the query peak Qi is not assigned to the key peak Kj, $X_{ij}=0$, and if the query peak Qi is assigned to the key peak Kj, $X_{ij}=1$, and $\text{shift}_{\text{query}}(i)$ and $\text{shift}_{\text{key}}(j)$ respectively represent chemical shifts of Qi and Ki,
(Constrains)
①

$$\sum_{i}^{M} X_{ij} \cdot \text{intg}_{\text{query}}(i) = \text{intg}_{\text{key}}(j)$$

,wherein $\text{intg}_{\text{query}}(i)$ is an integral value of the query peak Qi, and $\text{intg}_{\text{key}}(j)$ is an integral value of the key peak Kj
② If the multiplicity of Kj is different from the multiplicity of Qi, $X_{ij}=O$
③ If an integral value of Qi is greater than an integral value of Kj, $X_{ij}=0$
④ For two key peaks Ki_1 and Ki_2 having a COSY peak, there must be at least one COSY peak present between a set of all query peaks C_query(Ki_1) assigned to Ki_1 and a set of all query peaks C_query(Ki_2) assigned to Ki_2.

12. A system for estimating a molecular structure of a target substance, the system comprising:

a spectrum acquisition device that acquires a 1H-NMR spectrum and a 1H-1H NMR spectrum of a target substance from an NMR device as a key spectrum of the target substance;
a candidate molecular structure acquisition module that acquires candidate molecular structures of the target substance and calculates a candidate molecular structure list;
a virtual spectrum calculation module that calculates a virtual spectrum as a candidate spectrum from the candidate molecular structures included in the candidate molecular structure list;
a spectrum information extraction module that extracts spectrum information from each of the key spectrum and the candidate spectrum;
a spectrum similarity calculation module that calculates similarity between the key spectrum and the candidate spectrum by using the spectrum information of the key spectrum and the candidate spectrum; and
a final estimated structure output module that outputs a candidate molecular structure corresponding to a candidate spectrum with the highest similarity as a final estimated molecular structure of the target substance.

13. The system of claim 12, further comprising:

a hydrogen structural feature extraction module that identifies and extracts structural feature information of each hydrogen from each candidate molecular structure of the target substance;

a peak assignment validity determination module that verifies, based on the structural feature information of each hydrogen identified from the candidate molecular structure, whether each hydrogen of the candidate molecular structure may be assigned to all peaks in the spectrum; and

a valid candidate molecular structure output module that updates the candidate molecular structure list by using results of the peak assignment validity determination module.

14. The system of claim 12 or claim 13, wherein the spectrum information extracted from the spectrum information extraction module comprises the following information:

(a) Peak position: x-axis value of each peak in one-dimensional spectrum
(b) Peak split value: Number of small peaks forming each peak
(c) Peak integral value: Area value of each peak
(d) Cosy information (COSY): Information of all peak pairs in which cosy peaks are observed.

15. The system of claim 14, wherein the spectrum similarity calculation module calculates the similarity between the key spectrum and the candidate spectrum by using an objective function in Equation 5 below:

(Equation 5)

$$f = \sum_{i}^{M} \left( \sum_{j}^{N} \left( X_{ij} \cdot \left| shift_{query}(i) - shift_{key}(j) \right|^2 \right) \right)$$

M and N are respectively the number of peaks of the query spectrum and the key spectrum, X is a binary matrix of $M \times N$, and if the query peak Qi is not assigned to the key peak Kj, $X_{ij}=0$, and if the query peak Qi is assigned to the key peak Kj, $X_{ij}=1$, and $shift_{query}(i)$ and $shift_{key}(j)$ respectively represent chemical shifts of Qi and Ki,
(Constrains)
①

$$\sum_{i}^{M} X_{ij} \cdot intg_{query}(i) = intg_{key}(j)$$

,wherein $intg_{query}(i)$ is an integral value of the query peak Qi, and $intg_{key}(j)$ is an integral value of the key peak Kj
② If the multiplicity of Kj is different from the multiplicity of Qi, $X_{ij}=0$
③ If an integral value of Qi is greater than an integral value of Kj, $X_{ij}=0$
④ For two key peaks Ki_1 and Ki_2 having a COSY peak, there must be at least one COSY peak present between a set of all query peaks C_query(Ki_1) assigned to Ki_1 and a set of all query peaks C_query(Ki_2) assigned to Ki_2.

16. The system of claim 15, further comprising a deuterium substitution position calculation module that finds a Qi of a query spectrum of a final candidate molecular structure assigned to Kj in which is a singlet and a multiplet are mixed among the peaks of the key spectrum, and identifies a hydrogen of the corresponding final candidate molecular structure as a hydrogen substituted with a deuterium.

FIG. 1

**FIG. 2**

S10
ACQUIRE SPECTRUM

S20
ACQUIRE SPECTRUM INFORMATION

S30
ESTIMATE CANDIDATE MOLECULAR STRUCTURE

S40
TEST VALIDITY OF CANDIDATE MOLECULAR STRUCTURE

S41
EXTRACT HYDROGEN STRUCTURE FEATURE INFORMATION

S42
DETERMINE PEAK ASSIGNMENT POSSIBILITY

S43
UPDATE CANDIDATE MOLECULAR STRUCTURE

S50
ACQUIRE CANDIDATE SPECTRUM INFORMATION

S61
EXTRACT SPECTRUM COMPARISON INFORMATION

S62
CORRESPOND SPECTRUM PEAK

S63
CALCULATE SPECTRUM SIMILARITY

S60
COMAPRE SPECTRA

S71
DETERMINE FINAL CANDIDATE MOLECULAR STRUCTURE

S72
ESTIMATE DEUTERIUM SUSTITITION POSITION

S70
CONFIRM TARGET SUBSTANCE MOLECULAR STRUCTURE

FIG. 3

EP 4 510 139 A1

# EP 4 510 139 A1

| INTERNATIONAL SEARCH REPORT | International application No. |
|---|---|
| | **PCT/KR2024/000498** |

**A. CLASSIFICATION OF SUBJECT MATTER**

**G16C 20/40**(2019.01)i; **G16C 20/20**(2019.01)i; **G16C 20/70**(2019.01)i; **G16C 20/80**(2019.01)i; **G16C 20/90**(2019.01)i

According to International Patent Classification (IPC) or to both national classification and IPC

**B. FIELDS SEARCHED**

Minimum documentation searched (classification system followed by classification symbols)

G16C 20/40(2019.01); G01N 24/08(2006.01); G01R 33/46(2006.01); G01R 33/465(2006.01); G16C 20/20(2019.01); G16C 20/30(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1H-NMR, 1H-1H COSY, 2D NMR, 스펙트럼(spectrum), 피크 스플릿(peak split), 분자 구조(molecular structure), 유사도(similarity), 예측(prediction)

**C. DOCUMENTS CONSIDERED TO BE RELEVANT**

| Category* | Citation of document, with indication, where appropriate, of the relevant passages | Relevant to claim No. |
|---|---|---|
| A | US 2022-0383989 A1 (THE UNIVERSITY OF CHICAGO) 01 December 2022 (2022-12-01) See claims 1-6 and 15; and paragraphs [0080] and [0082]. | 1-16 |
| A | US 2022-0413072 A1 (REGENERON PHARMACEUTICALS, INC.) 29 December 2022 (2022-12-29) See claim 1. | 1-16 |
| A | US 2003-0229456 A1 (BEGER, Richard D. et al.) 11 December 2003 (2003-12-11) See abstract. | 1-16 |
| A | US 2015-0300968 A1 (NYMIRUM, INC.) 22 October 2015 (2015-10-22) See abstract. | 1-16 |
| A | US 2016-0131603 A1 (THE GEORGE WASHINGTON UNIVERSITY, A CONGRESSIONALLY CHARTERED NOT-FOR-PROFIT) 12 May 2016 (2016-05-12) See entire document. | 1-16 |

| ☐ Further documents are listed in the continuation of Box C. | ☑ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2024** | **18 April 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/000498**

| Patent document cited in search report | | | Publication date (day/month/year) | Patent family member(s) | | | Publication date (day/month/year) |
|---|---|---|---|---|---|---|---|
| US | 2022-0383989 | A1 | 01 December 2022 | WO | 2021-091883 | A1 | 14 May 2021 |
| US | 2022-0413072 | A1 | 29 December 2022 | WO | 2023-278443 | A1 | 05 January 2023 |
| US | 2003-0229456 | A1 | 11 December 2003 | US | 7996156 | B2 | 09 August 2011 |
| US | 2015-0300968 | A1 | 22 October 2015 | EP | 2929335 | A1 | 14 October 2015 |
| | | | | US | 10228337 | B2 | 12 March 2019 |
| | | | | US | 10522239 | B2 | 31 December 2019 |
| | | | | US | 2017-0032078 | A1 | 02 February 2017 |
| | | | | WO | 2014-089301 | A1 | 12 June 2014 |
| US | 2016-0131603 | A1 | 12 May 2016 | WO | 2014-204990 | A2 | 24 December 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HIDEYUKI MASUI** ; **HUIXIAO HONG**. Organic Chemistry. *J. Chem. Inf. Model.*, 2006, vol. 46, 2